# EUROPEAN PATENT APPLICATION

(11) **EP 1 803 822 A1**
(43) Date of publication of application: **04.07.2007**
(21) Application number: 05028761.4
(22) Date of filing: 30.12.2005
(51) Int. Cl.: C12Q 1/68

(54) **Method for the individual characterization of therapeutic target molecules and use thereof**

(71) Applicant: Adnagen AG, 30853 Hannover-Langenhagen (DE)
(72) Inventor: Böcher, Oliver, Dr., 8952 Schlieren-ZH (CH); Hollmann, Christiane, Dr., 30900 Wedemark (DE); Lankiewicz, Silke, Dr., 30853 Langenhagen (DE)
(74) Representative: Pfenning, Meinig & Partner GbR

(57) **Abstract**

The present invention concerns a method for the individual characterization of at least one therapeutic target molecule for tumor therapy in a sample taken from a patient, including the step of:
analyzing the mRNA of disseminated tumor cells for the expression of variants of said at least one therapeutic target molecule.

## Description

The present invention concerns a method for the individual characterization of at least one therapeutic target molecule for tumor therapy, a method for specification and/or individual decision on the method of treatment of an individual cancer patient, a method for treating an individual cancer patient, in particular for prevention of a primary tumor relapse and treatment of local or distant metastases in a patient.

The prognostic value of disseminated tumor cells (DTC, in particularly blood-borne DTC) has been proven repeatedly and provides a new predictive clinical tool for improvement of therapy and survival in cancer (Meng et al. (2004), HER-2 gene amplification can be acquired as breast cancer progresses. Proc. Natl. Acad. Sci. USA 101, 9393-9398).

In this document, Meng et al. analyzed disseminated tumor cells for gene amplification via FISH. They report, that the gene amplification state of disseminated tumor cells correlates with the status of metastases. However, this study and other histochemical studies of primary tumors often neglect the fact that in normal and tumor cells, different variants of therapy target genes (e.g. EGFR transcription and/or protein variants) can exist. These variants (naturally occurring through alternative splicing or in tumor cells through somatic mutations) exist e.g. in the whole EGFR sequence. Soluble proteins arise without cytosolic c-terminal ends as well as membrane anchored proteins without the n-terminal ligand-binding domain.

Today, two new classes of therapies exist exemplified for the case of EGFR: (1) antibodies that compete with the ligands of EGFR, for example EGF or TGF-α, and block the n-terminal binding domain of the receptor (Erbitux) and (2) small molecular weight receptor tyrosine kinase inhibitors (Iressa, Tarceva) that bind to the intracellular receptor tyrosine kinase domain and block the ATP binding site reversibly or irreversibly: Both therapies eliminate signal transduction of EGFR.

But the clinical experience shows that not all patients (about 20 %) respond to such therapies even if the primary tumor was tested positive for EGFR expression by IHC and/or EGFR gene amplification by FISH. On the other hand, patients which were tested negative with immunohistochemical methods sometimes respond to Erbitux therapy.

One reason could be that the DTC can change their expression profile during tumor progression. Another reason can be that not all DTC express the wild-type EGFR but some of the EGFR variants. Variants without the n-terminal binding domain (e.g. variant III) do not bind Erbitux. EGFR variants without somatic mutations in the receptor tyrosine kinase domain need higher doses of, for example Iressa, to block the whole signal transduction of the EGFR. Thus, Iressa or Tarceva have a high effect in NSCLC patients with single nucleotide mutations. Some new antibodies against different EGFR variants (e.g. EGFRvIII) are in pre-clinical studies.

Therefore, it is important to know which variant of a special therapy target gene is expressed in the DTC for providing patients the correct therapy. Because of tissue availability, the primary tumor is frequently used to establish the patient's EGFR status, but it is the metastases that are being treated with antibodies or receptor tyrosine kinase inhibitors. Another aspect of therapy is that some patients (e.g. with NSCLC)) become resistant to Iressa or Tarceva during tumor progression. The metastases of these patients often have or develop "second mutations" that are responsible for the resistance. But when the physician is able to diagnose the patients with resistant metastases it is often too late to change the therapy because re-biopsies are not routinely performed.

Beside partial sequence mutations of therapy targets, some tumors like breast or colorectal cancer develop gene amplification during tumor progression as detected by HER-2 or EGFR FISH analysis of the primary tumor. After the determination of the gene status, a Herceptin^{®} therapy is administered if the breast tumor was positive for HER-2. Because of the fact, that the rationale of Herceptin^{®} administration is entirely based on the HER-2 status of the primary tumor, patients with HER-2 negative primary tumors and concomitant HER-2 positive metastases do not benefit from Herceptin^{®} therapy. However, these patients would benefit from therapy, if HER-2 expression in DTC as analogue to the HER-2 status in distant metastases would be taken into account.

The present invention therefore aims to improve the knowledge about metastases and thereby support the decision about therapy of a patient. It is thus the object of the present invention, to provide a method with which, in a reliable and repeatable manner, the characterization of a tumor with respect to possible therapeutic target molecules can be improved.

This object is solved by the method for the individual characterization of at least one therapeutic target molecule according to claim 1 as well as by the method for stratification or individual decision on the method of treatment according to claim 27, the method for treating an individual cancer patient according to claim 28 and the use of those methods according to claim 31. Further improvements of the claimed methods are provided in the respective dependent claims.

According to the present invention, a method is provided, wherein at least one therapeutic target molecule is individually characterized by analyzing a sample taken from a patient for variants or mRNA of that at least one therapeutic target molecule. Most relevant to the present invention is the fact, that the mRNA expression of disseminated tumor cells within such a sample is analyzed.

The present application is based on the finding, that expression profiling of the DTC revealed strong evidence of tumor cell heterogeneity within different patients and even in the course of the disease within the same patient as is demonstrated in the examples shown below. Moreover, it is based on the finding, that the gene expression in DTC correlates with the histopathologically determined expression status in distant metastases, which can be different from the expression status in primary tumors. With the present method, it is in particular possible, to analyze the status of a possible metastases even if the primary tumor has already been removed by surgery or being treated by therapeutic agents.

Therapeutic target molecules may comprise proteins, peptides, mRNA, in particular tumor-associated cell surface antigens, growth-factors and their receptors, hormone receptors, transcription factors, cell cycle regulating proteins and cytoskeletal proteins as described in claim 24. Most important therapeutic target molecules for the present invention are members of the ErbB-family, the progesterone receptor (PR), the estrogen receptor (ER), mucin 1, tubulin, members of the human epithelial antigen EpCAM-family, members of the vascular endothelial growth factor (vEGF) family and the vEGF receptor (vEGFR) family and members of the platelet-derived growth factor (PDGF) family and the PDGF receptor (PDGFR) family, as described in claim 25. Variants to be analyzed in the present invention comprise among others variants in primary, secondary or tertiary structure of proteins and peptides, partial or total sequence deletions, in particular c-terminal or n-terminal deletions, single nucleotide mutations (SNPs), insertions, translocations, inversions and other genetic, transcriptional or translational variants.

The sample used in the inventive method may be e.g. a body fluid, bone marrow, lymph nodes or biopsies.

Further, the disseminated tumor cells (DTC) may be separated from the sample if necessary to improve the analysis, for example by positive or negative cell selection in liquid or solid phase. Suitable cell isolation methods comprise density gradient centrifugation, flow cytometry, micromanipulation, microdissection, e.g. with cells marked by fluorophores, or the method described in the international patent application PCT/EP02/05489 as published. The disclosure of this application with respect to the separation of disseminated tumor cells from a sample is fully included into the present application and further described within claims 8 to 21 of the present application.

The present invention is particularly useful for the stratification and individual characterization of the status of DTC for primary tumors and metastases of the colon, breast, lung or prostate. The inventive method may be used for prevention of a relapse of the primary tumor and/or for treatment of local and/or distant metastases of such primary tumor.

The present invention is particularly advantageous, because it is simple and easy as well as safe to take samples as described in claim 2 from a patient thus minimizing the risk for the patient. Such samples taken are a suitable material for analyzing DTC, as DTCs are usually present in such samples even after surgery of the primary tumor. It is possible to take a whole sample approach and analyzing the mRNA of the DTC in the whole sample or analyzing only the mRNA of DTC after separation of the DTC from other cells in the sample. By the inventive analysis of disseminated tumor cells, it is possible to decide on the effective therapy regimen with newly found therapeutic targets depending on the variability of these targets. Even if only one specific variant of target mRNA is detected, it is possible to decide specifically on the appropriate therapy in the presence of this variant.

Thus, the present invention is a valuable tool for improvement of the individual characterization as well as the individual therapy of a patient as the prognostic value of viable disseminated tumor cells is superior in detecting and determining the status of distant or local metastases in comparison to the histopathologically determined expression status or the gene amplification status of the primary tumor or disseminated tumor cells.

Within this application, the term "disseminated tumor cell" is equivalent to the terms "circulating tumor cells" or "micrometastases", which all have the same meaning of tumor cells disseminated from their tumor origin and possibly circulating, e.g. in the blood of patients.

Further, the term "ligand" within this application means all molecules that bind specifically to a specific target molecule. Ligands might be e.g. lectines, hormones, chemokines, cell activity modulating substances, cell adhesion molecules or the like.

### Cell selection

In the following, we describe the cell selection method according to PCT/EP02/05489, WO03/023057 A2, (equivalent to EP 1 409 727 A2).

In this method, firstly the DTC to be selected are marked by means of at least one or a combination of antibodies or antibody derivatives or by a bispecific antibody or antibody derivative. As a result, it is possible to mark, separate and hence concentrate, in particular the sought cells. This means that, in a first step, a preferably combined immunological detection or selection is effected. There is understood by antibody derivative in this application any type of altered antibody or antibody fragment which has a binding site, for example single-chain antibodies, antibody fragments, such as FAB fragments or recombinant antibodies. When "antibodies" are mentioned in the following, antibodies and/or antibody derivatives are always denoted.

In a second step, at least one marker, preferably a combination of markers, is then detected on a molecular-biological basis with a predefined combination of detection reagents, said markers being specific for the sought cells or being able to be found preferentially in the latter so that here again the sought cells are selected specifically. This therefore concerns here a preferably combined molecular-biological detection. The basic concept of this selection method is therefore to combine a detection via a combination of immunological parameters with a detection via a combination of molecular-biological parameters. Quite exceptional detection results are produced as a result, with which an advance is made into areas of detection which were not accessible previously. Therefore, concentrations of sought cells in blood samples down to one cell per 5 millilitres can even be detected.

A selection of the DTC precedes the detection of the markers via the binding of various antibodies to the sought cells, because the expression of special surface proteins differentiates cells of one type from cells of another type. Thus, for example the expression of special surface proteins differentiates tumor cells from non-transformed cells of this cell type.

Since the special pattern of the surface antigens for example in the case of tumor cells also differs from blood cell-typical patterns, tumor cells in the blood can be differentiated. In order to identify tumor cells, antibodies which specifically recognise these special surface proteins are used as tools. The specific antibody binding is exploited for various analysis and separation methods.

Due to the intensive binding of immunoglobulins, selected specially for this purpose, separation of the detected cells from non-detected cells is possible in addition to detection of cells via their surface epitope.

Instead of antibodies, any suitable specifier may be used to mark cells specifically.

Various separation principles are possible:

### 1. Separation principle based on liquid phase; e.g. flow cytometry:

For the flow-cytometric analysis, antibodies are coupled with fluorescence dyes. Cells pass a light source (laser) individually in a constant liquid flow. Upon illumination of the cells, the fluorescence dyes bound to the antibodies are excited and radiate light of specific wavelengths. The radiated light is detected and the measured signal is stored in a digital form. The light signal can be assigned to individual cells. The antibody-marked cell is detected thus and can now be separated from other cells. For separation, the cells are isolated into the smallest drops. After detection of the antibody-marked cell, the corresponding drop is directed into a collection receptacle. A concentration of this type can be effected for example by FACS flow cytometry. For example, concentrated cells with fluorescence-marked monoclonal antibodies against tumor-specific surface proteins are thereby incubated. The marked cells are washed twice with PBS and, subsequent thereto, 10⁷ cells are resuspended in 1 ml PBS. For the isolation of the tumor cells, a FACS vantage SE flow cytometer (Becton Dickinson) is used. Data recording, instrument control and data evaluation are effected via the CellQuest Software. The sorted cells are transferred into a 1.5 ml reaction vessel (filled with 1 ml PES). The RNA can then be isolated as described later.

### 2. Separation principle based on solid phase; e.g. magnetic separation

Antibodies are coupled to para-magnetic particles for the magnetic separation. After introduction of the para-magnetic particles into a magnetic field, the particles migrate in the magnetic field. During the movement in this magnetic field, cells to which these coupled antibodies are bound, are entrained and separated from other cells.

For cell detection by means of magnetic particles, antibodies are thus coupled to para-magnetic particles which have a defined number of chemically activated sites on their surface. Coupling methods are known for example from James P. Gosling, Solid-phase Concepts and Design, in: R.F. Masseyeff, W. H. Albert N. A. Stoines (eds), Methods of Immunobogical Analysis, Vol. 1, VCH Verlagsgesellschaft mbH, Weinheim, pp. 507-529. The specificity of the separation is determined via the specificity of the antibodies. A blood sample containing target cells is mixed with antibody-coupled magnetic particles; then particles and blood are moved relative to each other, for example by "overhead rotation" of samples situated in a closed container or by movement of the particles due to changing magnetic fields. Those target cells which are detected by an antibody bound to the solid phase and hence securely bound, follow the movement of the particles. It is possible as a result, when applying a magnetic field, to withdraw the particles with the cells bound thereon from the blood (e.g. onto the wall of the separation vessel). The blood which is target cell-depleted in this manner can be exchanged for other solutions, the cells separated via magnetic particles remaining in situ until switching off/removal of the magnetic field and being available far further applications.

Specific antibody mixtures can be used advantageously for the detection of the tumor cells. For example, a combination of the antibodies MOC-31 and Ber-EP4 is suitable for detecting intestinal tumor cells in the blood.

**Table 1: Antibody mixture**

| **Antigen** | **Clone** | **Concentration** |
|---|---|---|
| epith. rel. antigen | MOC-31 (Novocastra Co.) | 1,25 µl/10⁶ cells |
| epithelial antigen | Ber-EP 4 (DAKO Co.) | 0,924 µg/10⁶ cells |

By means of the antibody mixture in the preceding table 1, disseminated tumor cells are detected preferentially, however with high specificity. This is based on the preferential expression of specific surface proteins which differentiates cancer cells from other cells.

The selection method according to PCT/EP02/05489 is substantially based furthermore on the fact that cell markers in the blood of patients are detected not for instance at an immunological or enzymatic level but by the fact that a molecular-biological marker, for example mRNA (messenger ribonucleic acid) of sought cells in a sample, for example in a blood sample, is detected.

Since individual markers are expressed differently in a therapy-dependent manner, a combination of tumor markers is advantageously tested in order to detect all the tumor cells circulating in the blood. As a result, tumor cells can also be detected when the expression of a specific marker is relatively low in a patient or in an illness stage, which otherwise could lead to a putatively negative result. The use of markers comes up against limits however usually for the reason that mononuclear blood cells have a background expression ("illegitimate transcription") which impedes exact analysis.

The expression of the genes mentioned in table 2 is detected as a marker, for example of tumors. The detection can thereby be implemented for one or two markers or also for any number of these tumor markers in combination with each other.

**Table 2:**

| Gene or gene product | Gene | Alternative designation |
|---|---|---|
| Human carcinoma-associated antigen GA733-2 gene | GA733-2 | GA733.2 |
| Human epidermal growth factor receptor (EGFR) gene | EGFR | EGFR |
| Human carcinoembryonic antigen (CEA) gene | CEA | CEA |
| Homo sapiens mucin 1 (MUC1) | MUC1 | CA15-3 |
| Homo sapiens C-erb B2/neu protein (ERBB2) gene | HER-2/neu | HER-2 |
| Homo sapiens claudin 7 (CLDN7), mRNA | claudin7 (CLDN7) | claudin7 |
| Alkaline phosphatase, placenta-like (Nago isozyme), (Germ-cell alkaline phosphatase), (PLAP-like) | ALPPL2 (GCAP) | PLAP |
| Homo sapiens gastrin-releasing peptide receptor (GPPR) gene | GRPR | GRPR |
| Homo sapiens high-mobility group (nonhistone chromosomal protein isoform I-C (HMGIC), mRNA | HMGIC | HMGI-C |
| Homo sapiens gene for cytokeratin 20 | CK20 | CK2O |
| Human MAGE-3 antigen (MAGE-3) gene | MAGE-3 | MAGE-3 |
| Homo sapiens stanniocalcin 1 (STC 1) gene | Stanniocalcin 1 (STC1) | stanniocalcin |

As an alternative to the above-presented separation principles according to PCT/EP02/05489, also any other separation principles from the state of the art, which are based on marking cells with ligands, antibodies or other specifiers, can be used.

### Correlation of DTC and metastases

Following the extensive description of the preferred method for selection of disseminated tumor cells, an example is provided, which shows the correlation of HER-2-mRNA-expression in DTC with the immunohistochemical (IHC) HER-2-status in distant metastases. The growth factor receptor HER-2 is of special importance in breast cancer, because overexpression in primary tumors correlates with a shorter survival time/rate. Therefore, an expression of HER-2 is a prerequisite for Herceptin® therapy administration. Hence, the HER-2 expression in DTC was compared with the HER-2 status in primary tumors and distant metastases.

In this example, the expression of HER-2-mRNA in DTC was analyzed by means of RT-PCR and compared with the IHC- HER-2-status in distant metastases in two patients. This is shown in the following Table 3. This example shows on principle that DTCs can be analyzed for specific sequences of their mRNA.

**Table 3:**

| Patient number | IHC-HER-2 status in the primary tumor | Tumor cell detection | HER-2-expression DTC | IHC-HER-2 status in distant metastases |
|---|---|---|---|---|
| 1 | 0 | + | - | 1+ |
| 2 | 0 | + | + | 3+ |

The staining intensity of IHC was scored as 3+ (dark staining), 2+ (mid-range staining), 1+ (faint staining), or 0 (minimal or no staining). A staining of 3+ and 2+ is classified as positive whereas a staining of 1+ and 0 is classified as negative for the expression of HER-2.

In both patients, the HER-2 IHC status 0 was detected in the primary tumor. These patients were hence not treated with Herceptin®. In patient 1, the distant metastases examined showed a HER-2 status 1+ and DTC recovered from the same patient showed no mRNA expression of HER-2, illustrating a possible correlation of HER-2 expression in DTC and HER-2 status in distant metastases. In patient 2, this correlation was further substantiated since the distant metastases were staged 3+ for HER-2 and concomitant recovered DTC showed HER-2 expression.

In a further patient, we analyzed the HER-2 status in the primary tumor as well as in disseminated tumor cells selected from a peripheral blood sample. In the primary tumor, HER-2 was overexpressed three times whereas in the DTC no HER-2 expression could be detected. However, although a therapy according to the detection result for primary tumors was administered, the patient developed metastases. This example further shows that the expression-status of the primary tumor not necessarily correlates with the disseminated tumor cells detected in a blood sample and distant metastases.

### Correlation of DTC and primary tumor

In a further example, the expression of HER-2 mRNA in DTC was compared with histopathological data reflecting the HER-2 IHC status in the primary tumor (Table 4). In patients with HER-2 status 0 or 1+ (negative), DTC expressing no HER-2 mRNA were detected in 85 % of tumor cell positive blood samples, showing a positive correlation of HER-2 status in primary tumors and DTC. In patients with HER-2 status 2+ or 3+ (positive) in primary tumors, however, only 50 % of tumor cell positive patients showed a concomitant expression of HER-2 in DTC.

### Table 4:

### Tumor cell detection in metastasized breast cancer patients

The expression of the tumor-associated transcripts HER-2, MUC-1, GA 733-2 and Claudin-7 in disseminated tumor cells was analyzed by the disseminated tumor cells detection assay and compared with the HER-2 status in the primary tumor. Positive RT-PCR results are indicated by +, negative results by -. IHC status of HER-2 in primary tumor is indicated by 0 to 3+. "na" (not available) means that no histopathological data could be obtained.

| Patient Number | Tumor Cell Detection | Claudin-7 | MUC-1 | GA 733-2 | HER-2 | HER-2 Status of the Primary Tumor |
|---|---|---|---|---|---|---|
| 27 | + | + | + | + | + | 0 |
| 46 | + | + | + | + | + | 0 |
| 1 | - | - | - | - | - | 0 |
| 4 | - | - | - | - | - | 0 |
| 6 | - | - | - | - | - | 0 |
| 24 | - | - | - | - | - | 0 |
| 38 | - | - | - | - | - | 0 |
| 44 | - | - | - | - | - | 0 |
| 3 | + | - | + | - | - | 0 |
| 5 | + | + | + | + | - | 0 |
| 11 | + | + | - | + | - | 0 |
| 20 | + | + | - | - | - | 0 |
| 21 | + | - | - | + | - | 0 |
| 23 | + | - | - | + | - | 0 |
| 26 | + | + | - | + | - | 0 |
| 29 | + | + | + | + | - | 0 |
| 35 | + | + | + | + | - | 0 |
| 28 | - | - | - | - | - | 1+ |
| 15 | + | + | - | - | - | 1+ |
| 34 | + | + | + | + | - | 1+ |
| 8 | + | + | + | + | + | 3+ |
| 17 | + | + | - | - | + | 3+ |
| 36 | + | + | - | + | + | 3+ |
| 40 | + | + | + | + | + | 3+ |
| 41 | + | + | + | - | + | 3+ |
| 45 | + | - | - | - | + | 3+ |
| 2 | - | - | - | - | - | 3+ |
| 12 | - | - | - | - | - | 3+ |
| 13 | - | - | - | - | - | 3+ |
| 19 | - | - | - | - | - | 3+ |
| 32 | - | - | - | - | - | 3+ |
| 37 | - | - | - | - | - | 3+ |
| 7 | + | + | + | - | - | 3+ |
| 9 | + | + | - | - | - | 3+ |
| 18 | + | + | - | - | - | 3+ |
| 30 | + | + | + | - | - | 3+ |
| 31 | + | + | - | - | - | 3+ |
| 33 | + | + | - | - | - | 3+ |
| 39 | + | + | - | - | - | 3+ |
| 16 | + | + | + | + | + | na |
| 42 | + | + | + | + | + | na |
| 10 | - | - | - | - | - | na |
| 43 | - | - | - | - | - | na |
| 14 | + | + | + | - | - | na |
| 22 | + | + | + | + | - | na |
| 25 | + | + | + | - | - | na |
| 47 | + | + | + | - | - | na |
| 48 | + | + | + | + | - | na |

There was an 85% correlation between the HER-2 status 0 and 1+ of primary tumors and the absence of HER-2 mRNA expression in disseminated tumor cells. However, there was only a 50% correlation between the HER-2 status 2+ and 3+ in primary tumors and the presence of HER-2 mRNA expression.

Only patients with detectable disseminated tumor cells were included in these evaluations.

In patients with circulating tumor cells and with a HER-2 status 3+ in the primary tumor only 50% showed HER-2 expression in their disseminated tumor cells. This might be attributable to selective destruction of HER-2 expressing disseminated tumor cells through Herceptin and/or chemotherapy. This hypothesis is further supported by others who observed absence of disseminated tumor cells with HER-2 gene amplification after treatment with Herceptin and chemotherapy (Meng et al., PNAS Vol. 101 no. 25 (2004), pages 9393-9398).

Comparison of HER-2 expression in disseminated tumor cells with HER-2 status of distant metastases in breast cancer patients revealed 100% agreement between HER-2 expression in disseminated tumor cells and distant metastases, as HER-2 mRNA was detectable in disseminated tumor cells with a corresponding positive HER-2 status in metastasis (3+, patient 27). No HER-2 mRNA was detected in disseminated tumor cells with a corresponding negative HER-2 status 1+ in metastasis (patient 3). In both patients the primary tumor was staged 0 for HER-2, demonstrating an absolute correlation of HER-2 mRNA expression in disseminated tumor cells and HER-2 status in distant metastases and only a 50% correlation between disseminated tumor cells and primary tumors, i.e. disseminated tumor cells may differ substantially from tumor cells of the primary tumor with respect to the HER-2 status.

In the light of these results, it is reasonable to presume that disseminated tumor cells, conversed from a HER-2 negative to a HER-2 positive phenotype during cancer progression, may form distant HER-2 positive metastases which now themselves tend to disseminate HER-2 positive tumor cells into the blood circulation. In the absence of the HER-2 negative primary tumor which has been resected in all cases shortly after diagnosis, these cells are then detectable.

### Analysis of variants

In a further example, the expression-status of variants of EGFR was analyzed in 3 colon carcinoma cell lines, SW480 (cell line 1), HT29 (cell line 2) and CaCo-2 (cell line 3), in a bioanalyzer 2001. For this analysis, EGFR was amplified with primers T-566 (5'-AAACTGCACCTCCATCAGTG-3') and T-567 (5`-ATTCGTTGGACAGCCTTCAAG-3`) using HotStarTaq Mix (QIAGEN GmbH, Hilden, Germany) with the following protocol: 95 °C, 15 min, 30 cycles (94 °C, 30 s, 60 °C, 30 s, 72 °C, 1 min) and 72 °C, 5 min.

The EGFR amplificates were purified with QiAquick PCR Purification Kit (QIAGEN GmbH, Hilden, Germany) and the concentration was determined by a Bioanalyzer 2100 (Agilent Technologies, Waldbronn, Germany). For sequencing about 10 - 15 ng of PCR-fragments were processed with ABI PRISM BigDye Terminator Cycle Sequencing Ready Reaction Kit (Applera Deutschland GmbH, Darmstadt, Germany).

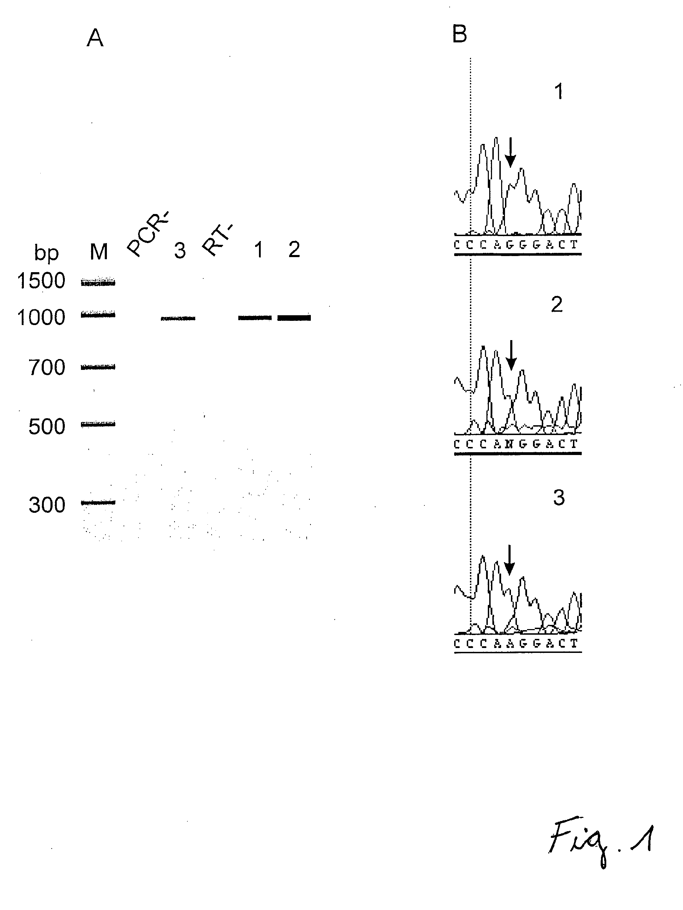

This analysis is shown in figure 1 wherein figure 1A shows the result for the 3 cell lines 1, 2 and 3 (lanes marked accordingly) on the bioanalyzer 2001. The lane marked with "M" contains a marker ladder to show the size of the amplificates. The lanes marked with "PCR" and "RT" show the result of a PCR-negative control sample and a sample without reverse transcription respectively. In figure 1B, the sequences of the purified EGFR-fragments for the 3 cell lines 1, 2 and 3 are shown. The arrows show different nucleotides at amino acid positions 497. The G to A nucleotide change is a natural occurring SNP resulting in two variants of EGFR.

This example analyzes three different cell lines, SW480, HT29, CaCo-2 and demonstrates that it is possible to detect sequence variants of EGFR in different tumor cells by the method of the present invention.

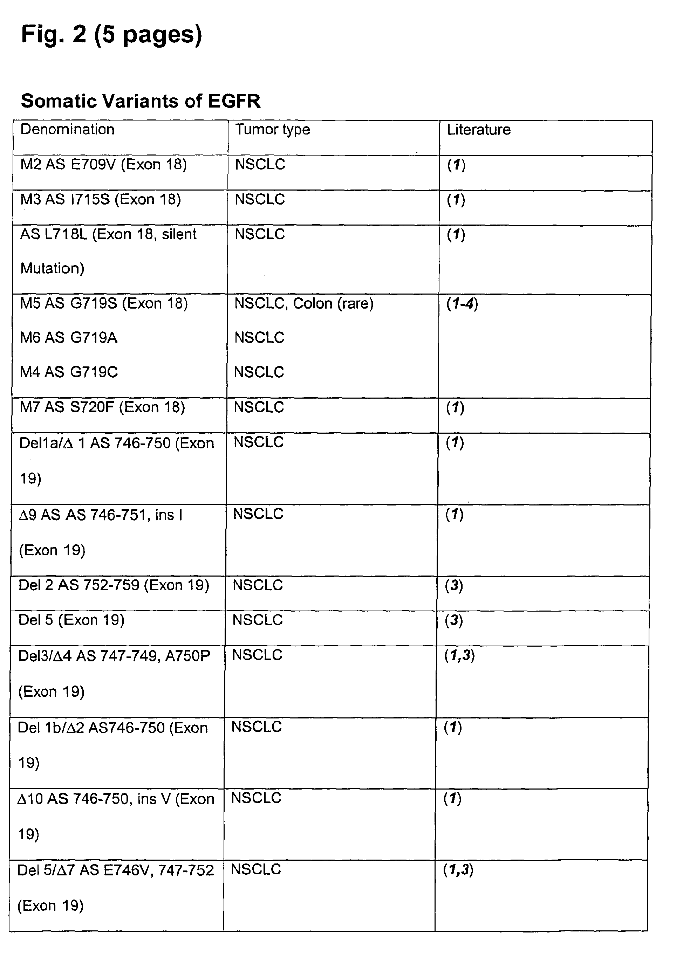

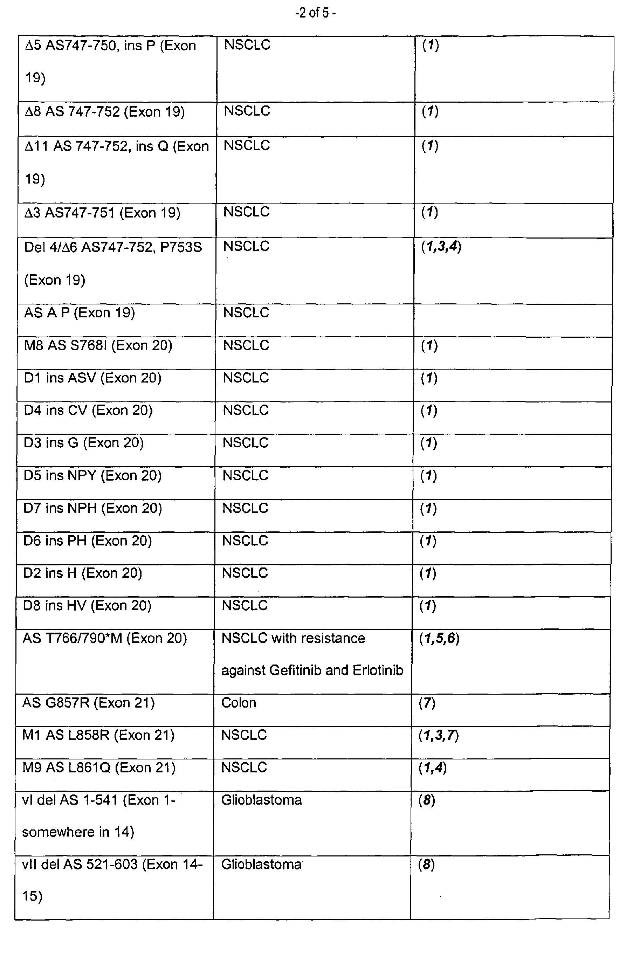

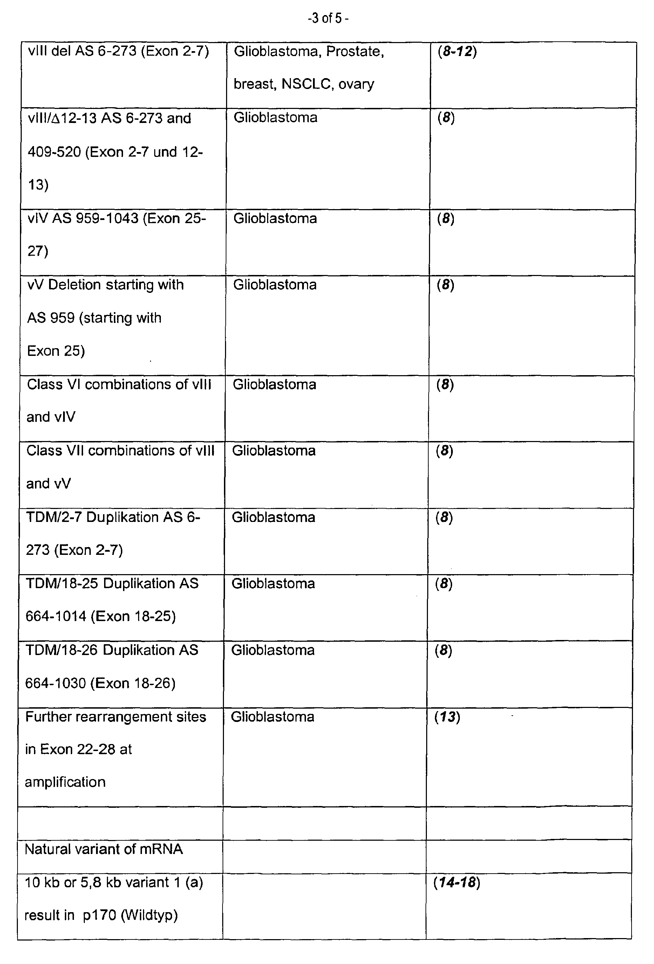

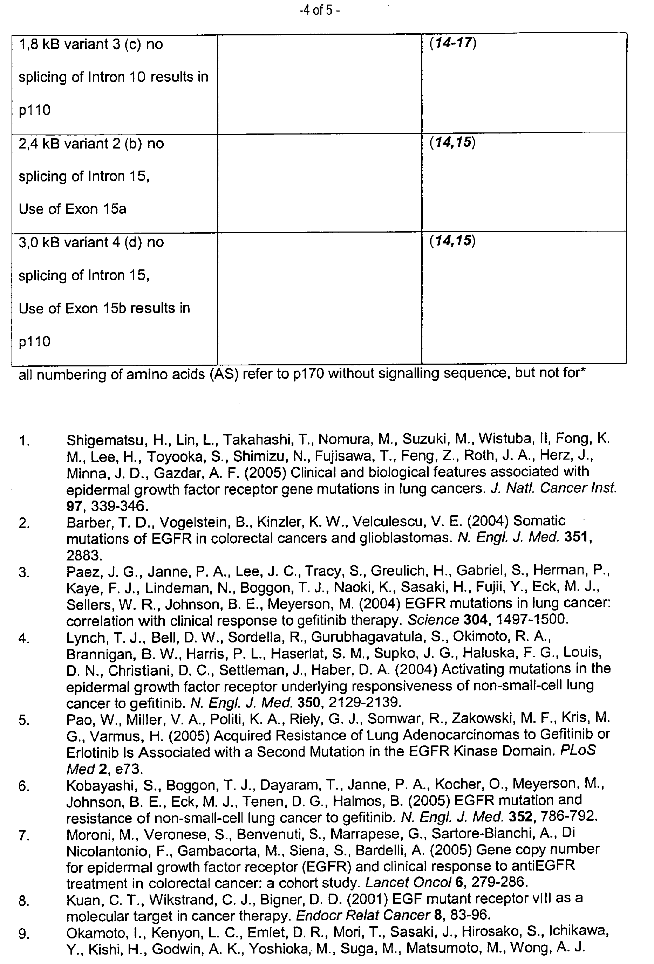

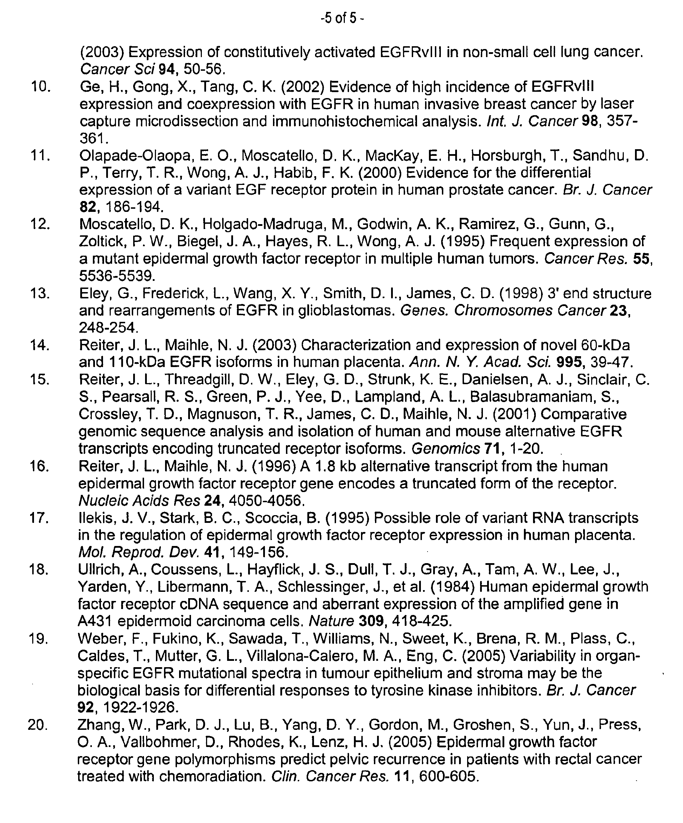

Further variants of EGFR which are relevant for the present invention are shown in figure 2 together with the respective prior art, wherein these variants are described.

To summarize, the present invention provides a new method for individual characterization of therapeutic target molecules for tumor therapy in a sample taken from a patient. By analyzing the mRNA of the tumor cells with respect to specific variants of the therapeutic target molecules, it becomes possible to easily and safely modify the therapy decision as well as the therapy itself in order to prevent a relapse of the primary tumor or preventing or treating local and/or distant metastases in a patient. The method according to claim 1 only contains steps outside of the human body dominated by the analysis of mRNA in disseminated tumor cells. Further, the present invention also comprises methods which concern the development and modification of therapy strategies as well as methods for therapeutically treating a patient.

## Claims

1. A method for the individual characterization of at least one therapeutic target molecule for tumor therapy in a sample taken from a patient, including the step of:
analyzing the mRNA of disseminated tumor cells for the expression of variants of said at least one therapeutic target molecule.

2. The method according to claim 1, wherein the sample comprises at least one of the following:
a body fluid, peripheral blood, sputum, ascites, lymph, urine, bone marrow, lymph nodes and biopsies.

3. The method according to claim 1 or 2, wherein disseminated tumor cells are isolated from the sample for use in the analyzing step.

4. The method according to claim 3, wherein the tumor cells are isolated by positive cell selection.

5. The method according to claim 3, wherein the tumor cells are isolated by one of negative cell selection and depletion of cells different than disseminated tumor cells.

6. The method according to one of claims 3 to 5, wherein the isolation of cells is effected in liquid phase or on a solid phase.

7. The method according to one of claims 3 to 6, wherein the disseminated tumor cells are isolated from the sample by density gradient centrifugation.

8. The method according to claim 4, wherein isolating the disseminated tumor cells from the sample includes the following steps:
mixing the sample with at least one specifier of tumor cell-associated markers and separating the cells marked with at least one of said at least one specifier.

9. The method according to claim 5, wherein isolating the disseminated tumor cells from the sample includes the following steps:
mixing the sample with at least one specifier of non-tumor cell-associated markers and separating the cells marked with at least one of said at least one specifier.

10. The method according to claim 8 or 9, wherein the sample is mixed with at least two specifiers.

11. The method according to one of claims 8 to 10, wherein at least one of the at least one specifier is coupled to solid phases in order to separate the cells from the sample.

12. The method according to one of claims 8 to 10, wherein at least one of the at least one specifier is marked with fluorophores and the separation of the marked cells from the sample is effected by means of flow cytometry, micromanipulation or microdissection.

13. The method according to one of claims 8 to 10, wherein at least one of the at least one specifier is coupled to magnetic or paramagnetic particles and
in order to separate the marked cells from the sample, the magnetic or paramagnetic specifier-coupled particles are separated magnetically from the sample after mixing with the sample.

14. The method according to claim 8, wherein the at least one of the at least one specifier has a binding site which binds to tumor cells of one or more tumor types or sub-types.

15. The method according to one of claims 8 to 14, wherein an antibody and/or ligand is used as specifier.

16. The method according to claim 8, wherein isolating the disseminated tumor cells from the sample includes the following steps:
mixing the sample with at least one antibody, that binds with its binding site to an epitope of disseminated tumor cells and isolating the cells marked with the at least one antibody from the sample.

17. The method according to claim 8, wherein isolating the disseminated tumor cells from the sample includes the following steps:
mixing the samples with (a) a predetermined combination of at least two antibodies and/or antibody derivatives, that bind with their binding sites to different epitopes of the cells to be isolated and/or with (b) at least one bispecific antibody an/or antibody derivative, that binds with its two binding sites to different epitopes of the cells to be isolated, wherein the binding sites bind to tumor cells, and
isolating the cells marked with at least one of the antibodies and/or antibody derivatives from the sample.

18. The method according to one of claims 1 to 17, wherein the disseminated tumor cells are analyzed for the presence or absence of at least one tumor-associated mRNA variant.

19. The method according to one of claims 1 to 18, wherein before or together with analyzing the mRNA of the disseminated tumor cells for the expression of variants of said at least one therapeutic target molecule, the separated tumor cells are analyzed with at least one molecular-biological reagent for the expression of at least one mRNA sequence, the expression of which is effected at least in disseminated tumor cells.

20. The method according to one of claims 1 to 18, wherein before or together with analyzing the mRNA of the disseminated tumor cells for the expression of variants of said at least one therapeutic target molecule, the separated tumor cells are analyzed with a predetermined combination of at least two molecular-biological detection reagents for at least two mRNA sequences for the expression of at least one mRNA sequence of said predetermined combination of at least two mRNA sequences, the expression of which is effected at least in disseminated tumor cells.

21. The method according to claim 19 or 20, wherein at least one of the mRNA sequences is associated with the expression of variants of said at least one target molecule.

22. The method according to one of claims 1 to 21, wherein at least segments of mRNA to be analyzed are amplified and/or detected using polymerase chain reaction (PCR), ligase chain reaction (LCR), nucleic acid sequence based amplification (NASBA), reverse transcription polymerase chain reaction (RT-PCR), linear amplification, transcription mediated amplification (TMA), loop-mediated isothermal amplification
(LAMP) and/or hybridization methods.

23. The method according to one of claims 1 to 22, wherein the therapeutic target molecule is a protein, peptide, mRNA or variants thereof.

24. The method according to one of claims 1 to 23, wherein the at least one therapeutic target molecule is one of the following: tumor-associated cell surface antigens, growth factors and their receptors, hormone receptors, transcription factors, cell cycle regulating proteins and cytoskeletal proteins.

25. The method according to claim 23, wherein the at least one therapeutic target molecule is one of the following: members of the ErbB-family, the progesterone receptor (PR), the estrogen receptor (ER), mucin 1, tubulin, members of the human epithelial antigen EpCAM-family, members of the vascular endothelial growth factor (vEGF) family and the vEGF receptor (vEGFR) family and members of the platelet-derived growth factor (PDGF) family and the PDGF receptor (PDGFR) family.

26. The method according to one of claims 1 to 25, wherein the variants are different in primary, secondary and/or tertiary structure, comprise partial or total sequence deletion variants, c-terminal or n-terminal deletion variants, single nucleotide variants, insertions, translocations and/or inversions.

27. A method for stratification and/or individual decision on the method of treatment of an individual cancer patient, wherein at least one therapeutic target molecule is individually **characterized** according to one of claims 1 to 26 and wherein it is individually decided on the method of treatment on the basis of the detected expressed variant or variants of said at least one therapeutic target.

28. Method of treating an individual cancer patient, wherein at least one therapeutic target molecule is individually **characterized** according to one of claims 1 to 26 and the patient is individually treated on the basis of the detected expressed variant or variants of said at least one therapeutic target.

29. Method according to claim 28, wherein when a variant of at least one therapeutic target is detected, which is susceptible to a medicament, said medicament is administered to the patient.

30. Use of a method according to one of claims 1 to 29 for the treatment of a primary tumor relapse and/or local or distant metastases in a patient.

31. Use of a method according to one of claims 1 to 30 for the treatment of a primary colon tumor, primary breast tumor, primary lung tumor and/or primary prostate tumor and/or metastases thereof.


SEQUENCE LISTING

<110> Adnagen AG

<120> Method for the individual characterization of
      therapeutic target molecules and use thereof

<130> 049EP1635

<140> 049EP1635
<141> 2005-12-30

<160> 2

<170> PatentIn Ver. 2.1

<210> 1
<211> 20
<212> DNA
<213> Artificial Sequence

<220>
<223> Description of Artificial Sequence: Primer

<400> 1
aaactgcacc tccatcagtg                                              20


<210> 2
<211> 21
<212> DNA
<213> Artificial Sequence

<220>
<223> Description of Artificial Sequence: Primer

<400> 2
attcgttgga cagccttcaa g                                            21